(19) Europäisches Patentamt
European Patent Office
Office européen des brevets

(11) Publication number: **0 075 377 A2**

(12) # EUROPEAN PATENT APPLICATION

(21) Application number: 82201427.0

(22) Date of filing: 01.07.80

(51) Int. Cl.[3]: **C 07 C 149/40**, C 07 C 147/11, C 07 C 147/14, C 07 C 101/54, C 07 C 65/21, C 07 C 69/92, A 01 N 41/10, A 01 N 37/40, A 01 N 37/44

(30) Priority: 18.07.79 GB 7925032
18.07.79 GB 7925033
08.11.79 GB 7938804
15.11.79 GB 7939634
12.02.80 GB 8004604

(43) Date of publication of application: 30.03.83 Bulletin 83/13

(84) Designated Contracting States: BE CH DE FR GB IT LI NL SE

(60) Publication number of the earlier application in accordance with Art. 76 EPC: 0023392

(71) Applicant: IMPERIAL CHEMICAL INDUSTRIES PLC, Imperial Chemical House Millbank, London SW1P 3JF (GB)

(72) Inventor: Griffin, David Alan, 12 Orion Roman Hill, Bracknell Berkshire (GB)
Inventor: Cartwright, David, 1 Stonehaven Drive Woodley, Reading Berkshire (GB)
Inventor: Cox, John Michael, 412A Finchampstead road, Wokingham Berkshire (GB)
Inventor: Collins, David John, 10 Ardwell Close, Crowthorne Berkshire (GB)

(74) Representative: Downes, John Edward et al, Imperial Chemical Industries PLC Legal Department: Patents Thames House North Millbank, London SW1P 4QG (GB)

(54) **Diphenyl ether compounds, their use as herbicides, and herbicidal compositions containing them.**

(57) Compounds of the formula (I):

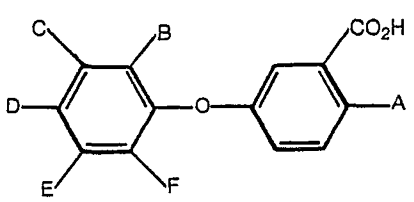

(I)

wherein A is hydroxy, lower alkoxy, mercapto, lower alkylthio, alkylsulphinyl or alkylsulphonyl, or lower mono- or dialkylamino, methyl, or trifluoromethyl; B is fluorine or chlorine; C and E are hydrogen; D is trifluoromethyl; and F is hydrogen, chlorine, or fluorine, and salts, esters and amides thereof. The compounds are useful as herbicides.

EP 0 075 377 A2

DIPHENYL ETHER COMPOUNDS, THEIR USE AS HERBICIDES, AND HERBICIDAL COMPOSITIONS CONTAINING THEM

This application is a divisional application based upon European Patent Application No. 80302211.0, the contents of which are herein incorporated by reference.

This invention relates to chemical compounds useful as herbicides, and to herbicidal compositions and processes utilising them.

According to the present invention, there are provided compounds of the formula (I)

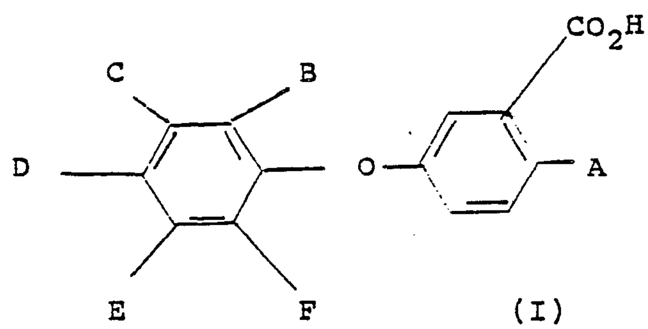

(I)

wherein A is hydroxy; alkoxy of 1 to 4 carbon atoms; mercapto; alkylthio, alkylsulphinyl, or alkylsulphonyl of 1 to 4 carbon atoms; mono- or di-alkylamino with alkyl groups of 1 to 4 carbon atoms; methyl; or trifluoromethyl;

B is fluorine or chlorine;
C is hydrogen;
D is trifluoromethyl;
E is hydrogen;

and F is hydrogen, chlorine, or fluorine; and salts, esters, and amides thereof.

Examples of compounds of the invention are listed in Table 1 below :

TABLE I

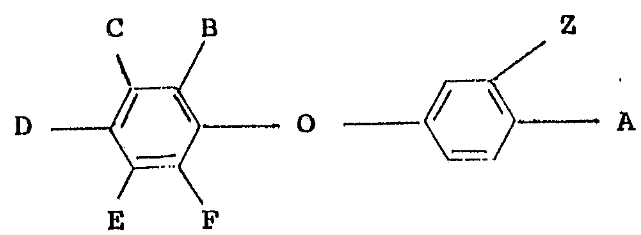

| COMPOUND NO | A | B | C | D | E | F | Z | MELTING POINT °C |
|---|---|---|---|---|---|---|---|---|
| 1 | $SC_2H_5$ | Cl | H | $CF_3$ | H | H | $CO_2H$ | 153-155 |
| 2 | SH | Cl | H | $CF_3$ | H | H | $CO_2CH_3$ | Oil |
| 3 | $SCH_3$ | Cl | H | $CF_3$ | H | H | $CO_2CH_3$ | Oil |
| 4 | $SOCH_3$ | Cl | H | $CF_3$ | H | H | $CO_2CH_3$ | 130 |
| 5 | $SO_2CH_3$ | Cl | H | $CF_3$ | H | H | $CO_2CH_3$ | 125 |
| 6 | $NHCH_3$ | Cl | H | $CF_3$ | H | H | $CO_2CH_3$ | 94 |
| 7 | OH | Cl | H | $CF_3$ | H | H | $CO_2H$ | 137 |
| 8 | $OCH_3$ | Cl | H | $CF_3$ | H | H | $CO_2CH_3$ | 73 |
| 9 | $N(CH_3)_2$ | Cl | H | $CF_3$ | H | H | $CO_2CH_3$ | 76 |
| 10 | $CF_3$ | Cl | H | $CF_3$ | H | H | $CO_2H$ | 81 |
| 11 | $CH_3$ | Cl | H | $CF_3$ | H | H | $CO_2H$ | 113 |

The carboxyl-substituted diphenyl ether derivatives (I) may be obtained by various synthetic methods. In one method, an appropriately substituted phenol may be reacted in salt form with an ester of an appropriately substituted chloro benzoic acid.

The ester so obtained may then be hydrolysed (e.g. by treatment with a mixture of acetic acid and aqueous hydrobromic acid) to the corresponding carboxylic acid (I).

In another method, 3-hydroxybenzoic acid or a 2-substituted-5-hydroxybenzoic acid may be reacted in the form of its di-salt with an appropriately substituted fluoro- or chlorobenzene, as described in Example 10.

The compounds of the invention are useful both as pre- and post-emergence herbicides. Pre-emergence herbicides are usually used to treat the soil in which a crop is to be planted, by application before or during seeding, or after seeding and before the crop emerges. Post-emergence herbicides are applied after the crop plants have emerged from the soil. Compounds of the invention may be used as selective herbicides in a variety of crops, including for example cotton, soya bean, peanuts, peas, wheat, sugar beet, barley and rice. Compounds of the invention may also be used as total herbicides. The compounds of the invention may be applied by any of the conventional techniques for applying herbicides. When applied as pre-emergence herbicides they may for example be sprayed on the surface of the soil before or during seeding, or after seeding and before emergence of the crop. In some situations for example in pre-emergence application to soya bean crops it may be advantageous to incorporate the compound of the invention into the soil before planting the crop. This may be done by applying the compound to the surface of the soil and then discing or harrowing the soil to mix the compound with the soil. Alternatively use may be made of the applicators which

have been developed for placing herbicides in a band beneath the surface of the soil.

In another aspect, therefore, the invention provides a process of killing or severely injuring unwanted plants, which comprises applying to the plants or to the locus thereof, a compound of the formula (II) or a salt thereof as hereinbefore defined.

As will be understood by those skilled in the art, the amount of the compound (II) applied will depend upon a variety of factors, for example the particular compound chosen for use and the identity of the unwanted plants. By way of general guidance, however, an amount of from 0.1 to 5.0 kilograms per hectare is usually suitable, while from 0.25 to 1.0 kilograms per hectare is preferred.

The compounds used in the process of the invention are preferably applied in the form of a composition, in which the active ingredient is mixed with a carrier comprising a solid or liquid diluent. In another aspect, therefore, the invention provides a herbicidaal composition, comprising as an active ingredient a compound of the formula (II) as hereinbefore defined, in admixture with a solid or liquid diluent. Preferably the composition also comprises a surface-active agent.

The solid compositions of the invention may be for example, in the form of dusting powders, or may take the form of granules. suitable solid diluents include, for example, kaolin, bentonite, kieselguhr, dolomite, calcium carbonate, talc, powdered magnesia, and Fuller's earth. Solid compositions also include soluble powders and granules which may comprise a salt of a compound of the invention in admixture with a water-soluble carrier, or a mixture of a compound of the invention with an alkali for example sodium or potassium carbonate; when mixed with water the composition gives a solution of a salt of the compound of the invention.

Solid compositions may also be in the form of

dispersible powders or grains comprising in addition to the active ingredient, a wetting agent to facilitate the dispersion of the powder or grains in liquids. Such powders or grains may include fillers, suspending agents and the like.

Liquid compositions include aqueous solutions, dispersions and emulsions containing the active ingredient preferably in the presence of one or more surface active agents. Water or organic liquids may be used to prepare solutions, dispersions, or emulsions of the active ingredient. The liquid compositions of the invention may also contain one or more corrosion inhibitors for example lauryl isoquinolinium bromide.

Surface active agents may be of the cationic, anionic or non-ionic type. Suitable agents of the cationic type include for example quaternary ammonium compounds, for example cetyltrimethyl ammonium bromide. Suitable agents of the anionic type include for example soaps, salts of aliphatic mono-esters of sulphuric acid, for example sodium lauryl sulphate; and salts of sulphonated aromatic compounds, for example dodecylbenzene-sulphonate, sodium, calcium and ammonium lignosulphonate, butylnaphthalene sulphonate, and a mixture of the sodium salts of diisopropyl- and triisopropyl-naphthalenesulphonic acid. Suitable agents of the non-ionic type include, for example, the condensation products of ethylene oxide with fatty alcohols such as oleyl alcohol and cetyl alcohol, or with alkyl phenols such as octyl-phenol, nonylphenol, and octylcresol. Other non-ionic agents are the partial esters derived from long chain fatty acids and hexitol anhydrides, for example sorbitol monolaurate; the condensation products of the said partial esters with ethylene oxide and the lecithins.

The compositions which are to be used in the form of aqueous solutions, dispersions or emulsions are generally supplied in the form of a concentrate containing

a high proportion of the active ingredient, the concentrate being diluted with water before use. These concentrates are usually required to withstand storage for prolonged periods and after such storage to be capable of dilution with water in order to form aqueous preparations which remain homogeneous for a sufficient time to enable them to be applied by conventional spray equipment.

The compositions of the invention may contain, in addition to carriers and surface-active agents, various other constituents to increase their usefulness. They may contain, for example, buffering salts to maintain the pH of the composition within a desired range; antifreeze agents, for example urea or propylene glycol; adjuvants, for example oils and humectants; and sequestrants, for example citric acid and ethylenediaminetetracetic acid, which help to prevent the formation of insoluble precipitates when the compositions are diluted with hard water. Aqueous dispersions may contain antisettling agents and anti-caking agents. The compositions may in general contain a dye or pigment to impart a characteristic colour. Agents for increasing viscosity may be added to reduce the formation of fine droplets during spraying, and thereby reduce spray drift. Other additives useful for particular purposes will be known to those skilled in the formulation art.

In general concentrates may conveniently contain from 10 to 85% and preferably from 25 to 60% by weight of active ingredient. Dilute preparations ready for use may contain varying amounts of the active ingredient, depending upon the purpose for which they are to be used; however, dilute preparations suitable for many uses contain between 0.01% and 10% and preferably between 0.1% and 1% by weight of the active ingredient.

The invention is illustrated by the following Examples, in which all parts are by weight and all temperatures in degrees Centigrade unless otherwise stated.

## EXAMPLE 1

This Example illustrates a method of preparing 2-ethylthio-5-(2-chloro-4-trifluoromethylphenoxy)benzoic acid (Compound No. 1 of Table I).

2-Iodo-5-(2-chloro-4-trifluoromethylphenoxy) benzoic acid (0.1 mole) in dimethylformamide (500 ml) was cooled to $0^{o}$. Cuprous oxide (0.11 mole) was added, followed by ethyl mercaptan (0.1 mole). The mixture was stirred while sodium hydride (0.25 mole) was added in portions. After 15 minutes the temperature was raised to $80^{o}$ for 6 hours. The mixture was cooled and poured into hydrochloric acid (2 molar). The mixture was twice extracted with ether. The ether extract was washed with water, dried ($Na_2SO_4$) and evaporated. The residue was recrystallised from ether to give 2-ethylthio-5-(2-chloro-4-trifluoromethylphenoxy)benzoic acid, with a melting point of $153-155^{o}$.

## EXAMPLE 2

This Example illustrates a method of preparing methyl 2-mercapto-4-(2-chloro-4-trifluoromethylphenoxy) benzoate (Compound No. 2 of Table I).

Methyl 2-diazo-5(2-chloro-4-trifluoromethylphenoxy) benzoate tetrafluoborate (15.0 g, prepared as described in Example 10) was added portionwise at $30^{o}$ to a stirred solution of tetramethylthiourea (4.0 g) in acetonitrile (50 ml). The mixture was stirred for a further forty-five minutes at $50^{o}$, an excess of sodium iodide added to decompose any remaining diazonium salt and the solvent removed in vacuo. The residue was triturated many times, first with water, then ether. The oil was dissolved in dichloromethane, dried and evaporated to give a solid salt.

This salt (3.65 g) was stirred with N sodium

hydroxide solution until homogeneous (approximately thirty minutes). The solution was extracted with ether, acidified with 2N hydrochloric acid and again extracted with ether. The latter extracts were washed with water and brine, dried and evaporated to give methyl 2-mercapto-5-(2-chloro-4-trifluoromethylphenoxy) benzoate (1.11 g).

## EXAMPLE 3

This Example illustrates a method of preparing methyl 2-methylthio-5-(2-chloro-4-trifluoromethylphenoxy) benzoate (Compound No. 3 of Table I).

A solution of methyl 2-mercapto-5(2-chloro-4-trifluoromethylphenoxy)benzoate (1.76 g, prepared as described in Example 2) in N,N-dimethylacetamide (10 ml) was treated with sodium hydride (0.24 g, 50%, prewashed with petroleum b.p. 40-60$^{o}$). The mixture was stirred for ten minutes, treated with methyl iodide (0.5 ml, excess) and stirred for a further fifteen minutes. It was then diluted with water and extracted with ether. The extracts were washed with water and brine, dried and evaporated to give methyl 2-methylthio-5-(2-chloro-4-trifluoromethylphenoxy)benzoate.

## EXAMPLE 4

This Example illustrates a method of preparing methyl 2-methylsulphinyl-5-(2-chloro-4-trifluoromethylphenoxy) benzoate (Compound No. 4 of Table I).

A solution of methyl 2-methylthio-5(2-chloro-4-trifluoromethylphenoxy) benzoate (1.55 g) in methanol (50 ml) was treated with sodium metaperiodate (0.9 g) in water (10 ml). The mixture was heated at 80$^{o}$ for one hour, cooled, diluted with water and extracted with ethyl acetate. The extracts were washed with water, dried, evaporated and the residue chromatographed on silica in

ether to give methyl 2-methylsulphinyl-5-(2-chloro-4-trifluoromethylphenoxy) benzoate (0.65 g, mp 130°).

EXAMPLE 5

This Example illustrates a method of preparing methyl 2-methylsulphonyl-5-(2-chloro-4-trifluoromethylphenoxy) benzoate (Compound No. 5 of Table I).

A solution of methyl 2-methylthio-5(2-chloro-4-trifluoromethylphenoxy) benzoate (1.55 g) and m-chloroperbenzoic acid (3.42 g, excess) in dichloromethane (50 ml) was refluxed for four hours, cooled, diluted with dichloromethane and washed with sodium bicarbonate solution, water and brine. The extracts were dried, evaporated and triturated with petroleum (b.p. 40-60°) to give methyl 2-methylsulphonyl-5-(2-chloro-4-trifluoromethylphenoxy) benzoate (1.1 g, m.p. 125°).

EXAMPLE 6

This Example illustrates methods of preparing Compounds 6 and 9 of Table I.

A mixture of methyl 2-amino-5(2-chloro-4-trifluoromethylphenoxy) benzoate (3.0 g) and dimethylmethylphosphonate (10 ml) was heated at 120° for four hours. It was then cooled, diluted with water, basified with 2N sodium hydroxide solution and extracted with ether. The extracts were washed with water and brine, dried and evaporated in vacuo to give a brown oil. Trituration with methanol gave methyl 2-methylamino-5-(2-chloro-4-trifluoromethylphenoxy) benzoate (Compound No. 6 of Table I) (0.49 g, m.p. 94°).

A similar experiment was carried out with the exception that the reaction period was extended to twenty hours at 170°. The mixture was cooled, diluted with

water and sodium hydroxide solution and extracted with ethyl acetate. The extracts were washed with water and brine, dried and evaporated in vacuo. The residue was triturated with methanol to give methyl 2-dimethylamino (2-chloro-4-trifluoromethylphenoxy) benzoate (Compound No. 9 of Table I) (1.83 g, m.p. 76°). This compound was also prepared by reductive alkylation. Thus treatment of the amine (0.43 g) with formaldehyde (40%, 1.5 ml), sodium cyanoborohydride (0.25 g) and acetic acid (0.13 ml) gave rise to 0.30 g of crude material contaminated with approximately 10% of methyl 2-methylamino-5-(2-chloro-4-trifluoromethylphenoxy) benzoate.

## EXAMPLE 7

This Example illustrates a method of preparing methyl 2-hydroxy-5-(2-chloro-4-trifluoromethylphenoxy) benzoate (Compound No. 7 of Table I).

2,5-Dihydroxybenzoic acid (1.68 g) was added portionwise to a stirred suspension of sodium hydride (1.68 g, 50%, prewashed with petroleum b.p. 40-60°) in N,N-dimethylacetamide (50 ml). The mixture was stirred for a further thirty minutes, then treated with 3-chloro-4-fluorobenzotrifluoride* (2.24 g). It was then heated at 120° for one hour, cooled, diluted with water, acidified with 2N hydrochloric acid and extracted with ethyl acetate. The extracts were washed with water and brine, dried and evaporated in vacuo. The residue was triturated with petroleum (b.p. 40-60°) and the resulting solid recrystallized from petroleum (b.p. 80-100°) to give Compound No. 7 (1.55 g, m.p. 137°).

* 3,4-Dichlorobenzotrifluoride can also be employed but the period of reaction is extended to thirteen hours at 150° and purification more complex.

A mixture of this material (1.35 g), methanol (25 ml) and boron trifluoride etherate (2 ml) was refluxed for twelve hours, treated with a further portion of the catalyst (1 ml) and refluxing continued for six hours more. Water was then added and the mixture extracted with ether. The extracts were washed with water and brine, dried, evaporated and the residue recrystallized from methanol to give the methyl ester of Compound No. 7 (1.04 g, m.p. 75°).

EXAMPLE 8

This Example illustrates a method of preparing methyl 2-methoxy-5-(2-chloro-4-trifluoromethylphenoxy)benzoate. (Compound No. 8 of Table I).

Methyl 2-hydroxy-5(2-chloro-4- trifluoromethylphenoxy)benzoate (0.35 g, prepared as above was added portionwise to a stirred suspension of sodium hydride (0.05 g, 50%, prewashed with petroleum b.p. 40-60°) in dimethylacetamide (5 ml). The mixture was stirred for a further thirty minutes, treated with methyl iodide (0.1 ml, excess), and heated at 60° for one hour. It was then diluted with water and extracted with ether. The extracts were washed with water and brine, dried and evaporated and the residue recrystallized from petroleum (b.p. 40-60°) to give Compound No. 8 of Table I (250 mg, m.p. 73°).

EXAMPLE 9

This Example illustrates the preparation of Compound No.11 of Table I.

A mixture of sodium hydride (2.3g, 50% dispersion in mineral oil, prewashed with petroleum, b.p. 40-60°) and 4-hydroxy-2-methylbenzoic acid (3.4g) in dry N,N-dimethylformamide (30ml) was stirred for thirty minutes,

then treated with 3-chloro-4-fluorobenzotrifluoride (4.5g). The mixture was heated at 130° for five hours, cooled, diluted with water, acidified with 2N hydrochloric acid and extracted with ethyl acetate. The extracts were washed with water and brine, dried and evaporated. The residue was recrystallised from petroleum (b.p. 80-100°) to give 2-methyl-5(2-chloro-4-trifluoromethylphenoxy)-benzoic acid (Compound No. 11 of Table I), (2.06g, m.p. 113°).

## EXAMPLE 10

This Example illustrates the preparation of 5-(2-chloro-4-trifluoromethylphenoxy)-2-trifluoromethylbenzoic acid (Compound 10 of Table I).

Finely powdered methyl 2-amino-5(2-chloro-4-trifluoromethylphenoxy)benzoate (16.5 g) was suspended in fluoboric acid (120 ml, 40%), treated with ethanol (100 ml) and cooled to 0°. A solution of sodium nitrite (4.5 g) in water (20 ml) was added dropwise to the stirred reaction mixture. After a further fifteen minutes at 0°, the crystalline precipitate was filtered off and washed with ether to give the diazonium fluoborate (12.32 g).

Sodium iodide (4.5 g) was added to a stirred solution of the diazonium salt (11.7 g) in acetonitrile (100 ml). After stirring at 20° for fiften minutes, effervescence had ceased. The solvent was removed in vacue and the residue partitioned between dichloromethane and water. The extracts were washed with water, dried and evaporated. The crude product was flash chromatographed on silica in ether to give 10.2 g of a mixture of methyl 2-iodo-5(2-chloro-4-trifluoromethylphenoxy)benzoate and its 2-H analogue (80:20 by GLC).

This material (2.0 g) was heated in a sealed Teflon lined pressure vessel for eighteen hours at 150° together with trifluoromethyl iodide (5 ml), acetonitrile (15 ml), dry pyridine (5 ml) and dry, freshly precipitated, copper powder (2.5 g). The crude reaction mixture was filtered

through Supercel, evaporated in vacuo, dissolved in ether, washed with 2N hydrochloric acid and water, dried and chromatographed on silica in ethyl acetate/petroleum, b.p. 60-80° (1:3). Pure methyl 2-trifluoromethyl-5(2-chloro-4-trifluoromethylphenoxy)benzoate (600 mg.), was obtained together with material contaminated with 2H-derivative. Rechromatography of the latter gave an additional 250 mg of pure material.

The 2-$CF_3$ ester (1.62 g) was treated with potassium hydroxide (230 mg), water (3 ml) and methanol (40 ml) and stirred at 60° for six hours. The mixture was cooled, diluted with water, extracted with ether to remove unchanged ester, acidified with 2N hydrochloric acid and extracted with ethyl acetate. The latter extracts were washed with water and brine, dried and evaporated and the residue chromatographed on silica gel in dichloromethane to give an oil which crystallized slowly on standing to a white solid 1.08 g, m.p. 81° identified as 5-(2-chloro-4-trifluoromethylphenoxy)-2-trifluoromethylbenzoic acid (Compound No. 10 of Table I).

## EXAMPLE 11

This Example illustrates the herbicidal properties of compounds of Table I. The compounds were submitted to herbicide tests as described below.

Each compound was formulated for test by mixing an appropriate amount of it with 5ml of an emulsion prepared by diluting 160ml of a solution containing 21.1 grams per litre of Span 80 and 78.2 grams per litre of Tween 20 in methylcyclohexanone to 500 ml with water. Span 80 is a Trade Mark for a surface-active agent comprising sorbitan monolaurate. Tween 20 is a Trade Mark for a surface-active agent comprising a condensate of 20 molar proportions of ethylene oxide with sorbitan monolaurate. The mixture of the compound and the emulsion was then shaken with glass beads and diluted

to 40 ml with water. The spray composition so prepared was sprayed on to young pot plants (post-emergence test) of the species named in Table IV below, at a rate equivalent to 1000 litres per hectare. Damage to plants was assessed 14 days after spraying by comparison with untreated plants, on a scale of 0 to 5 where 0 is 0 to 20% damage and 5 is complete kill. In the table of results, a dash (-) means that no test was made.

A test was also carried out to detect pre-emergence herbicidal activity. Seeds of the test species were placed on the surface of fibre trays of soil and were sprayed with the compositions at the rate of 1000 litres per hectare. The seeds were then covered with further soil. Three weeks after spraying, the seedlings in the sprayed fibre trays were compared with the seedlings in unsprayed control trays, the damage being assessed on the same scale of 0 to 5.

The results of the tests are given in Table IV below.

TABLE IV

| COMPOUND NO | RATE OF APPLICATION kg/ha | PRE- OR POST-EMERGENCE APPLICATION | TEST PLANTS Sb | Rp | Ct | Sy | Mz | Ww | Rc | Sn | Ip | Am | Pi | Ca | Po | Xa | Ab | Cv | Ot/Av | Dg | Pu | St | Ec | Sh | Ag | Cn |
|---|---|---|---|---|---|---|---|---|---|---|---|---|---|---|---|---|---|---|---|---|---|---|---|---|---|---|
| 10 | 1.0 | Pre | 5 | 4 | 0 | 4 | 1 | 0 | 0 | 4 | 3 | 5 | 5 | 5 | - | 0 | 4 | - | 0 | 4 | - | 4 | 2 | 1 | 1 | - |
| | | Post | 3 | 5 | 3 | 3 | 4 | 2 | 0 | 5 | 4 | 5 | 3 | 5 | - | 4 | 3 | - | 2 | 3 | - | 5 | 4 | 5 | 0 | 2 |
| 10 | 4.0 | Pre | 5 | 5 | - | 3 | 1 | 3 | 2 | 5 | 3 | 5 | 5 | 5 | - | 1 | 5 | - | 2 | 4 | - | 4 | 4 | 4 | 3 | - |
| | | Post | 4 | 5 | 4 | 4 | 5 | 3 | 1 | 5 | 4 | 5 | 4 | 4 | - | 4 | 5 | - | 4 | 5 | - | 5 | 4 | 5 | 3 | 2 |
| 3 | 5.0 | Pre | 4 | 1 | 4 | 1 | 0 | 0 | 0 | 3 | 3 | 5 | 5 | 1 | - | 2 | 1 | - | 0 | 2 | - | 3 | 0 | 4 | 0 | - |
| | | Post | 4 | 4 | 3 | 3 | 4 | 1 | 1 | 5 | 4 | - | 4 | 5 | - | 4 | 4 | - | 2 | 5 | - | 4 | 3 | 4 | 1 | - |
| 5 | 5.0 | Pre | 2 | 2 | 1 | 0 | 1 | 1 | 0 | 2 | 3 | 3 | 2 | 3 | - | 0 | 2 | - | 0 | 2 | - | 3 | 3 | 2 | 0 | - |
| | | Post | 3 | 3 | 2 | 3 | 3 | 0 | 0 | 5 | 3 | - | 3 | 4 | - | 1 | 3 | - | 2 | 2 | - | 4 | 2 | 2 | 0 | - |
| 6 | 5.0 | Pre | 1 | 0 | 0 | 0 | 0 | 0 | 0 | 3 | 1 | 3 | - | 0 | - | 0 | 0 | - | 0 | 0 | - | 0 | 0 | 0 | 0 | - |
| | | Post | 2 | 2 | 2 | 3 | 1 | 0 | 0 | 5 | 4 | 4 | - | 4 | - | 3 | 4 | - | 0 | 2 | - | 3 | 0 | 1 | 0 | - |
| 7 | 5.0 | Pre | 0 | 1 | 0 | 0 | 0 | 0 | 0 | 0 | 0 | 0 | - | 0 | - | 0 | 1 | - | 0 | 0 | - | 0 | 0 | 0 | 0 | - |
| | | Post | 1 | 3 | 1 | 4 | 3 | 1 | 0 | 2 | 2 | 3 | - | 3 | - | 1 | 2 | - | 0 | 2 | - | 3 | 1 | 2 | 0 | - |

Names of test plants in Table IV

| | |
|---|---|
| Sb | Sugar beet |
| Rp | Rape |
| Ct | Cotton |
| Sy | Soya bean |
| Mz | Maize |
| Ww | Winter wheat |
| Rc | Rice |
| Sn | *Senecio vulgaris* |
| Ip | *Ipomoea purpurea* |
| Am | *Amaranthus retroflexus* |
| Pi | *Polygonum aviculare* |
| Ca | *Chenopodium album* |
| Po | *Portulaca oleracea* |
| Xs | *Xanthium spinosum* |
| Ab | *Abutilon theophrasti* |
| Cv | *Convolvulus arvensis* |
| Ot/Av | Oats (cultivated in pre-emergence test and *Avena fatua* (wild oats) in post-emergence test) |
| Dg | *Digitaria sanguinalis* |
| Pu | *Poa annua* |
| St | *Setaria viridis* |
| Ec | *Echinochloa crus-galli* |
| Sh | *Sorghum halepense* |
| Ag | *Agropyron repens* |
| Cn | *Cyperus rotundus* |

JED/jlw
DIV 4
SPEC293

1. Compounds of the formula (I)

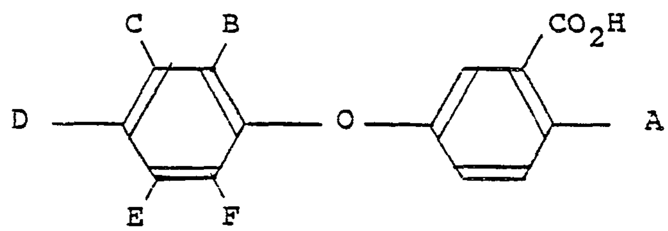

(I)

wherein A is hydroxy; alkoxy of 1 to 4 carbon atoms; mercapto; alkylthio, alkylsulphinyl, or alkylsulphonyl of 1 to 4 carbon atoms; mono- or dialkylamino with alkyl groups of 1 to 4 carbon atoms; methyl; or trifluoromethyl;
B is fluorine or chlorine;
C is hydrogen;
D is trifluoromethyl;
E is hydrogen;
and F is hydrogen, chlorine, or fluorine
and salts, esters, and amides thereof.

2. Herbicidal compositions comprising as an active ingredient at least one compound of formula (I) as claimed in claim 1, in admixture with a carrier comprising a solid or liquid diluent.

3. A process of inhibiting the growth of unwanted plants, which comprises applying to the plants, or to the locus thereof, a herbicidal amount of at least one compound of the formula (I) as defined in claim 1.